# EUROPEAN PATENT SPECIFICATION

(11) **EP 0 378 165 B1**
(45) Date of publication and mention of the grant of the patent: **04.05.1994**
(21) Application number: 90100364.0
(22) Date of filing: 09.01.1990
(51) Int. Cl.: C07C 29/132, C07C 33/20

(54) **Method for producing aromatic alcohol**
Verfahren zur Herstellung eines aromatischen Alkohols
Procédé pour la préparation d'un alcool aromatique

(30) Priority: 13.01.1989 JP 4862/89; 08.12.1989 JP 317613/89
(43) Date of publication of application: 18.07.1990
(73) Proprietor: MITSUBISHI PETROCHEMICAL CO., LTD., Chiyoda-ku Tokyo (JP)
(72) Inventor: Inaba, Masashi, c/o Yokkaichi Research Center, 1, Toho-cho, Yokkaichi-shi, Mie-ken (JP); Hamana, Ryozo, c/o Yokkaichi Research Center, 1, Toho-cho, Yokkaichi-shi, Mie-ken (JP); Hase, Hideyuki, c/o Yokkaichi Research Center, 1, Toho-cho, Yokkaichi-shi, Mie-ken (JP); Ashizawa, Tatsuro, c/o Kashima Division, Kamisu-machi, Kashima-gun, Ibraki-ken (JP)
(74) Representative: Kolb, Helga, Dr. Dipl.-Chem.

(56) References cited:
- FR-A- 1 345 953
- FR-A- 2 207 892
- JOURNAL OF CATALYSIS, vol. 29, 1973, pages 367-373; J.H.R. CASEMIER et al.:"The oxidation of cumene and the decomposition of cumene hydroperoxide onsilver, copper, and platinum"
- PATENT ABSTRACTS OF JAPAN, vol. 10, no. 194 (C-358)[2250], 8th July 1986; & JP-A-61 38 631
- PATENT ABSTRACTS OF JAPAN, vol. 8, no. 102 (C-222)[1539], 12th May 1984; & JP-A-59 16 843

## Description

This invention relates to a process for the production of aromatic alcohols using improved catalysts.

Aromatic alcohols are useful as intermediates for various types of organic compounds and as solvents, and are advantageously produced industrially by the reduction of aromatic hydroperoxides.

Japanese Patent Publication No.26961/1964 and U.S. Patent No.2,491,926 disclose methods of producing α-cumyl alcohol by reducing cumene hydroperoxide or dicumyl peroxide dissolved in cumene with hydrogen in the presence of hydrogenation catalysts such as Pd, Ni, etc.

Further, in order to prevent eluation and deactivation over time of catalyst for preparing aromatic alcohols, a method is disclosed in Japanese Patent Laid-open No. 38631/1986, wherein a catalyst or carrier thereof is water repellant.

Such reactions are accompanied by the evolution of heat; therefore, use of a solvent is recommended for mitigating reaction heat and for lowering side reactions as much as possible. As said solvents, water-immiscible solvents such as hydrocarbons are employed. However, deactivation of the catalyst is recognized to occur unexpectedly soon when said solvents are used, so, a method of employing a lower aliphatic alcohol is disclosed by Japanese Patent Laid-open No.69527/1980. Further, in order to obtain aromatic alcohols in good yield a method is disclosed in Japanese Patent Laid-open No.174737/1985, wherein said reduction is conducted in coexistence with amines or some compounds capable of being converted to amines during said hydrogenation reaction.

However, in the hydrogenation method described in said Patent Laid-open, a complex process is necessary for the separation of desired aromatic alcohols from aliphatic alcohols, amines, etc. having different properties from those of the aromatic alcohols. In addition, a complex structure of equipment is needed for the separation of the produced alcohols and a suspended catalyst,and therefore, the method mentioned above can not be advantageously employed for industrial mass production thereof.

Japanese Patent Laid-open No.16843/1984 discloses, when reducing aromatic hydroperoxide with hydrogen gas in the presence of a catalyst containing Pd, a method wherein a feed liquid containing aromatic hydroperoxide is allowed to pass downstream in a fixed bed reactor. Japanese Patent Laid-open No.110639/1984 discloses, when reducing aromatic hydroperoxide with hydrogen, a method wherein a Pd catalyst with Pd surface area of at least 200m²/g Pd is employed in the form of a fixed bed.

It is described that in the hydrogenation method in said patent Laid-open elution of the Pd was barely found, and that the desired aromatic alcohols can be rather effectively obtained. The operating time reported in said patent Laid-open was only 720 hours (30 days), whereas, the present inventors conducted an even longer time of operation to find a completely deactivated catalyst at the 70th day of operation (refer to COMPARATIVE EXAMPLE 3 below). In addition, when we measured the concentrations of Pd in the liquid produced by passing aromatic hydroperoxide solutions having concentrations changed over the Pd catalyst, we recognized a proportional relationship between both concentrations as shown in the next TABLE 1.

**TABLE 1**

| Concentration of cumene hydroperoxide in feed (weight by %) | Concentration of metal in solution produced (concentrated 200 times) (weight by ppm) |
|---|---|
| | Pd(0.3%)/γ-alumina catalyst |
| 2.0 | 7 |
| 4.0 | 14 |
| 8.3 | 32 |
| 16.1 | 46 |

Test liquids were passed at 60°C, and at a LHSV of 6/hour.

That is, as not insignificant quantities of Pd were eluted with aromatic hydroperoxide, the use of a Pd catalyst results in the problem of the inevitable deactivation of the catalyst.

When using a supported Ni catalyst, the catalyst has a disadvantage in spite of having a capacity to reduce aromatic hydroperoxide with hydrogen, of giving a low yield of the desired aromatic alcohols because of many side-reactions (refer to COMPARATIVE EXAMPLE 4 below).

It is an object of the present invention, therefore, to provide a method improved with respect to the catalysts of producing aromatic alcohols wherein no problems such as stated above will occur.

### SUMMARY OF THE INVENTION

This invention in outline, relates to a method of producing aromatic alcohols, that is, a method of manufacturing aromatic alcohols by reducing aromatic hydroperoxides with hydrogen in liquid phase which comprises using a catalyst containing Pt and at least one element selected from the group consisting of Pb, Sn, Cu, As, Sb, In, Se and Bi in a fixed bed reactor.

The present inventors, as a result of earnest investigations conducted to overcome said problems, have found that by using a Pt catalyst in a fixed bed reactor in a method of reducing aromatic hydroperoxides with hydrogen in liquid phase, the catalyst will not be deactivated, but rather will achieve high and stable activity even during long periods of use.

Also even though a liquid containing aromatic hydroperoxide was passed over a supported Pt catalyst in accordance with the conditions in TABLE 1, the Pt concentration (after 200-fold concentration) of the produced liquid did not exceed the limit of detection (1 wt.ppm) and no Pt was found in the production liquid, and it was confirmed that the elution of Pt due to aromatic hydroperoxide hardly occured when a Pt catalyst was used, to complete the present invention.

However, even though the use of a Pt catalyst in a fixed bed reactor may stabilize higher activity, it was determined that as far as slight amounts of impurities were concerned, a part of the produced aromatic alcohol was further converted by aromatic ring hydrogenation to alcohol having a cyclohexane ring (refer to COMPARATIVE EXAMPLE 1 below). The quantity of this product obtained by aromatic ring hydrogenation is very small, but the boiling point thereof is very near to that of the aromatic alcohol making said product difficult to be removed, resulting in the problem of lowered product purity. Therefore, it is required to develop a catalyst which has long stable hydrogenation activity with less aromatic ring hydrogenation, when producing aromatic alcohols by reducing aromatic hydroperoxides.

The present inventors, for solving said problem, have earnestly conducted investigations and as a result, found that a stable higher activity can be obtained by use of said combined catalyst without accompanying aromatic ring hydrogenation.

### DETAILED DESCRIPTION OF THE INVENTION

The present invention will be concretely illustrated below.

### (Aromatic hydroperoxides)

As examples of aromatic hydroperoxides to be subjected to the hydrogenation reaction in this invention, alpha phenylethyl hydroperoxide, cumene hydroperoxide, cymene hydroperoxide, o,m- or o,p-diisopropylbenzene monohydroperoxide, o,m- or o,p-diisopropylbenzene dihydroperoxide, isopropylnaphathalene hydroperoxide, etc., and compositions which contain at least one member selected from the group consisting thereof can be given.

### (Hydrogen)

The amount of hydrogen to be fed to the hydrogenation reaction system in this invention is 1-50 times as much as the amount theoretically required, preferably 1-30 times and most preferably 1-20 times. Excess feeding of hydrogen causes loss thereof, and reqiures an undue scale of equipment in recovery and circulating systems, and sometimes gives rise to unnecessary side-reactions.

### (Catalyst)

The catalyst components used in the process of producing aromatic alcohols according to this invention are usually supported on suitable carriers. The Pt metal supporting ratios thereof are generally 0.01-5wt.%, preferably 0.03-3wt.%, wherein the ratio of the elements other than Pt is usually 0.001-3wt.%, preferably 0.005-2wt.%.

As for the carriers, heat-resistant inorganic compounds, for example, synthetic gels such as alumina, silica, etc., or natural inorganic carriers such as diatomaceous earth, porous clay, carbon, etc. can be given.

### (Reaction mode)

As for the reaction modes in the process of producing aromatic alcohols according to this invention, as shown in Japanese Patent Laid-open No.73709/1979, batch process, continuous process or any other suitable process which is well known as a hydrogenation method for organic peroxide can be embodied. However, when a catalyst is employed in a form of suspended bed, a catalyst removal process is needed, making its apparatus complicated; therefore, a fixed bed is preferable. In a fixed bed, when an upflow is used, the catalyst is liable to become fluidized to cause deactivation; therefore, a downflow is more preferable.

### (Solvent)

As for solvents used for diluting the aromatic hydroperoxides in the method of producing aromatic alcohol according to this invention, any solvent which is capable of solving aromatic hydroperoxides along with the products obtained therefrom can be used, and for example, aliphatic hydrocarbons, aromatic hydrocarbons and aromatic alcohols can be given. For instance, in a process of manufacturing cumene hydroperoxide cumen is present in said process as a solvent, and it can be used as a solvent as it is in a hydrogenation of cumene hydroperoxide in the process of the present invention. In addition it is also a preferable method to recycle cumyl alcohol as shown in a following formula, which is obtained by the hydrogenation of cumene hydroperoxide, to be used as a solvent.

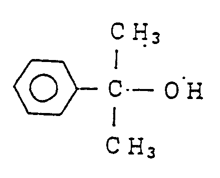

### (Concentration of aromatic hydroperoxide)

In the hydrogenation reaction using a catalyst in the process of producing aromatic alcohols according to this invention, it is desirable to control the concentration of aromatic hydroperoxide in the feed liquid supplied to the reactor to at most 25wt.%, preferably 0.01-15wt.%, and most preferably 0.1-10wt.%. When said concentration is more than 25wt.%, a considerable amount of heat is evolved, causing some problems such as hard control of reaction temperature, depression of high activity, formation of side reactions, etc.

### (Reaction temperature)

The hydrogenation reaction, in the method of producing aromatic alcohols according to this invention, is usually conducted in a temperature range of 20-120°C, preferably 30-120°C, and most preferably 40-120°C. Too high a reaction temperature is not desirable, because side reactions such as a decomposition reaction of the aromatic hydroperoxide itself or the like will violently take place. On the other hand too low a reaction temperature will produce problems such as decreased reaction rate, etc.

### (Reaction pressure)

The total pressure in the hydrogenation reaction in the method of preparing aromatic alcohols according to this invention, ranges usually from normal pressures to a certain high pressure, preferably from normal pressures to 49 bars (50kg/cm²G) and most preferably from normal pressures to 29.4 bars (30kg/cm²G). As the hydrogenation reaction proceeds easily, excess reaction pressure just entails excessive apparatus costs for no real effect, and causes problems such as subsequent hydrogenation reaction of the produced liquid and solvent.

### EXAMPLES

This invention will be further illustrated in detail with reference to the following examples. In the examples, % is meant to be weight % unless notified to the contrary.

### CATALYST PREPARATION EXAMPLE-1

After impregnating a 0.8% aqueous solution of hydrogen hexachloroplatinate (IV) (hexahydrate) into pellet type γ-alumina with dimensions of 3mmφ×3mm, the resultant pellets were dried at 110°C for 12 hours.

Then, the dried pellets were reduced in a hydrogen stream at 400°C for 16 hours.

Further impregnating a 0.1% aqueous solution of lead nitrate into the reduced Pt catalyst and after drying it at 110°C for 12 hours it was reduced in a hydrogen stream at 400°C for 16 hours to obtain a catalyst having a composition of Pt(0.3%)-Pb(0.03%)/γ-Al₂O₃.

### EXAMPLE-1

A 200 ml autoclave equipped with inlet pipes for raw material and hydrogen and an outlet pipe for liquid products and a basket type of stirrer filled with 0.8g of catalyst prepared according to CATALYST PREPARATION EXAMPLE-1 was maintained at 60°C. 1.2L/hour of solution of 3.5% cumenehydroperoxide (hereinafter abbreviated as CHP) in cumene and 12L/hour of hydrogen were fed therein continuously, while the hydrogenation products were continuously collected through the outlet pipe to keep the content in the autoclave at about 80ml. At this time the hydrogern pressure was kept at 7.5kg/cm²-G and the stirrer was rotated at 750rpm.

The results are shown in TABLE 2 below.

In addition one liter of catalyst prepared according to CATALYST PREPARATION EXAMPLE-1 was charged in a stainless steel tube with an inner diameter of 27.2mm equipped with a thermowell tube having an outer diameter of 6mm. keeping the temperature at the entrance of the catalyst layer at 45°C; 7.2L/hr. of liquid having a composition of 3.5% CHP, 77.0% cumyl alcohol and 19.5% cumene; and 72L/hr. of hydrogen at a pressure of 8kg/ cm²G were continuously fed thereto.

The conversion of CHP and selectivity to cumyl alcohol and 2-cyclohexyl-2-propanol (nuclear hydrogenation product of cumyl alcohol) after 8 hours from the start of feed are shown in TABLE 2 together with other examples.

### CATALYST PREPARATION EXAMPLES 2-8

Except for using in place of the aqueous solution of lead nitrate in CATALYST PREPARATION EXAMPLE-1, 0.1-1.0% solutions of stannous chloride (CATALYST PREPARATION EXAMPLE-3), cupric chloride (CATALYST PREPARATION EXAMPLE-4), arsenic trichloride [dioxane solution] (CATALYST PREPARATION EXAMPLE-5), antimony trichloride (CATALYST PREPARATION EXAMPLE-6), indium chloride (CATALYST PREPARATION EXAMPLE-7), selenium monochloride [benzene solution) (CATALYST PREPARAION EXAMPLE-8), and bismuth nitrate (CATALYST PREPARATION EXAMPLE-9) were used to prepare catalysts according to the method described in CATALYST PREPARATION EXAMPLE-1, comprising 0.3% of Pt, 0.3% of Sn and In or 0.03% of Cu, As, Sb, Se and Bi respectively supported on γ-alumina.

### EXAMPLE 2-8

The procedure of EXAMPLE-1 was repeated except that the catalysts prepared in CATALYST PREPARATION EXAMPLES 2 through 8 were used. The results are shown in TABLE 2 below.

### COMPARATIVE EXAMPLE 1

The procedure of EXAMPLE-1 was repeated except that a catalyst containing 0.3% Pt was used. The results are shown in TABLE 2.

The above catalyst was prepared as follows:

After impregnating a 0.8-1.3% aqueous solution of hydrogen hexachloroplatinate (IV) (hexahydrate) into pellet type γ-alumina with dimensions of 3mmφ×3mm, the resultant pellets were dried at 110°C for 12 hours.

Then, the dried pellets were reduced in a hydrogen stream at 400°C for 16 hours to obtain a supported Pt catalyst having a composition of Pt(0.3-0.5%)/γ-Al₂O₃.

### COMPARATIVE EXAMPLE-2

The procedure of EXAMPLE-1 was repeated except that the catalyst of Pt(0.3%)-Ag(0.03%)/γ-alumina prepared according to CATALYST PREPARATION EXAMPLE-1 using silver nitrate in place of lead nitrate was used. The results are shown in TABLE 2.

**TABLE 2**

| EXAMPLE | Additive element | Autoclave is used | Fixed bed downflow type reactor is used | | |
|---|---|---|---|---|---|
| | | Reaction rate of hydrogenation [mol/kg catalyst·hr] | Convertion of CHP [%] | Selectivity to cumyl alcohol [%] | Selectivity to 2-cyclohexyl-2-propanol [%] |
| EXAMPLE 1 | Pb | 61.8 | 99.9 | 99.0 | 0.34 |
| EXAMPLE 2 | Sn | 58.1 | 99.9 | 99.2 | 0.01 |
| EXAMPLE 3 | Cu | 57.5 | 99.8 | 99.1 | 0.26 |
| EXAMPLE 4 | As | 61.0 | 99.8 | 99.1 | 0.18 |
| EXAMPLE 5 | Sb | 57.7 | 99.9 | 99.2 | 0.02 |
| EXAMPLE 6 | In | 59.5 | 99.9 | 99.2 | 0.02 |
| EXAMPLE 7 | Se | 61.9 | 99.9 | 99.1 | 0.11 |
| EXAMPLE 8 | Bi | 61.3 | 99.8 | 99.0 | 0.24 |
| COMPARATIVE EXAMPLE 1 | -- | 61.6 | 99.8 | 98.7 | 1.04 |
| COMPARATIVE EXAMPLE 2 | Ag | 0.9 | 2.0 | 97.0 | 0.01 |

### COMPARATIVE EXAMPLE-3

After impregnation of a 0.6% aqueous solution of palladium chloride into pellet type γ-alumina with dimensions of 3mmφ×3mm, the resultant pellets were dried at 110°C for 12 hours.

Then, the dried pellets were reduced in a hydrogen stream at 400°C for 16 hours to obtain a supported Pd catalyst having a composition of Pd (0.3%)/γ-Al₂O₃.

The procedure of EXAMPLE-1 was repeated except that the Pd catalyst obtained in such a manner was used. The conversions of CHP after 8 hours and 20 days from the start of feed were 99.9% and 100% respectively, and the conversions after 40 and 70 days were 98.8% and 97.1% respectively, catalytic activity significantly decreasing.

### COMPARATIVE EXAMPLE-4

The procedure of EXAMPLE-1 was repeated except that the commercially available Ni catalyst [Trade name: Ni-3266E by Harshaw Co.] was used and the Ni catalys was pretreated in a hydrogen stream at 250°C for 4 hours prior to feed. The conversion of CHP after 8 hours from the start of feed was 97%, the yield of cumyl alcohol was as low as 81% and by-products as acetophenone, α-methylstyrene, 1-phenylethanol, etc. were found.

As can be understsood from the above, the manufacturing methods according to this invention permit stable hydrogenation of aromatic hydroperoxide with a high conversion rate as well as the production of corresponding aromatic alcohol with high selectivity.

## Claims

1. A method for producing an aromatic alcohol by reducing an aromatic hydroperoxide with hydrogen in a liquid phase characterized by using a catalyst comprising Pt and at least one element selected from the group consisting of Pb, Sn, Cu, As, Sb, In, Se and Bi in a fixed bed reactor.

2. A method according to claim 1, wherein the catalyst consists of Pt and Sn.

3. A method according to claim 1, wherein the catalyst consists of Pt and Sb.

4. A method according to claim 1, wherein the catalyst consists of Pt and In.

5. A method according to any one of claim 1 to 4, wherein the catalyst is supported on γ-alumina.

6. A method according to claim 5, wherein 0.01 to 5 weight % of Pt and 0.001 to 3 weight % of at least one element selected from the group of consisting of Pb, Sn, Cu, As, Sb, In, Se and Bi are supported.

7. A method according to claim 1, wherein the aromatic hydroperoxide is cumene hydroperoxide.

8. A method according to claim 1, wherein a concentration of the aromatic hydroperoxide in a feed is 0.01 to 15 weight %.

9. A method according to claim 1, wherein hydrogen reduction is carried out at the temperature ranging from 20 to 120°C and under the pressure ranging from normal pressure to (50 kg/ cm²G) 49 bars.

## Patentansprüche

1. Verfahren zur Herstellung eines aromatischen Alkohols durch Reduktion eines aromatischen Hydroperoxids mit Wasserstoff in einer flüssigen Phase, **gekennzeichnet** durch Verwendung eines Katalysators, der Pt und mindestens ein Element, ausgewählt aus der Gruppe bestehend aus Pb, Sn, Cu, As, Sb, In, Se und Bi, umfaßt, in einem Festbettreaktor.

2. Verfahren gemäß Anspruch 1, dadurch **gekennzeichnet**, daß der Katalysator aus Pt und Sn besteht.

3. Verfahren gemäß Anspruch 1, dadurch **gekennzeichnet**, daß der Katalysator aus Pt und Sb besteht.

4. Verfahren gemäß Anspruch 1, dadurch **gekennzeichnet**, daß der Katalysator aus Pt und In besteht.

5. Verfahren gemäß einem der Ansprüche 1 bis 4, dadurch **gekennzeichnet**, daß der Katalysator γ-Aluminiumoxid-gestützt ist.

6. Verfahren gemäß Anspruch 5, dadurch **gekennzeichnet**, daß 0,01 bis 5 Gew.% Pt und 0,001 bis 3 Gew.% mindestens eines Elements ausgewählt aus der Gruppe bestehend aus Pb, Sn, Cu, As, Sb, In, Se und Bi auf dem Träger aufgebracht sind.

7. Verfahren gemäß Anspruch 1, dadurch **gekennzeichnet**, daß das aromatische Hydroperoxid Cumenhydroperoxid ist.

8. Verfahren gemäß Anspruch 1, dadurch **gekennzeichnet**, daß die Konzentration des aromatischen Hydroperoxids im Zulauf 0,01 bis 15 Gew.% beträgt.

9. Verfahren gemäß Anspruch 1, dadurch **gekennzeichnet**, daß die Reduktion mit Wasserstoff bei der Temperatur im Bereich von 20 bis 120°C und unter einem Druck von Normaldruck bis 49 bar (50 kg/cm² G) ausgeführt wird.

## Revendications

1. Procédé pour la préparation d'un alcool aromatique en réduisant un hydroperoxyde aromatique avec de l'hydrogène dans une phase liquide, caractérisé par l'utilisation d'un catalyseur comprenant Pt et au moins un élément choisi dans le groupe constitué de Pb, Sn, Cu, As, Sb, In, Se et Bi dans on réacteur à lit fixe.

2. Procédé selon la revendication 1, dans lequel le catalyseur est constitué de Pt et Sn.

3. Procédé selon la revendication 1, dans lequel le catalyseur est constitué de Pt et Sb.

4. Procédé selon la revendication 1, dans lequel le catalyseur est constitué de Pt et In.

5. Procédé selon l'une quelconque des revendications 1 à 4, dans lequel le catalyseur est supporté sur de la γ-alumine.

6. Procédé selon la revendication 5, dans lequel O,O1 à 5 % en poids de Pt et O,OO1 à 3 % en poids d'au moins un élément choisi dans le groupe constitué de Pb, Sn, Cu, As, Sb, In, Se et Bi sont supportés.

7. Procédé selon la revendication 1, dans lequel l'hydroperoxyde aromatique est l'hydroperoxyde de cumène.

8. Procédé selon la revendication 1, dans lequel la concentration de l'hydroperoxyde aromatique dans une charge est de O,O1 à 15 % en poids.

9. Procédé selon la revendication 1, dans lequel la réduction avec de l'hydrogène est effectuée à une température comprise entre 2O et 12O°C sous une pression comprise entre la pression normale et (50 kg/cm²G) 49 bars.
